# EUROPÄISCHE PATENTSCHRIFT

(11) **EP 0 700 279 B1**
(45) Veröffentlichungstag und Bekanntmachung des Hinweises auf die Patenterteilung: **08.05.2002**
(21) Anmeldenummer: 94916139.2
(22) Anmeldetag: 27.05.1994
(51) Int. Cl.: A61H 31/00

(54) **VORRICHTUNG ZUR UNTERSTÜTZUNG DER HERZFUNKTION**
DEVICE FOR SUPPORTING THE FUNCTIONING OF THE HEART
DISPOSITIF D'ASSISTANCE A LA FONCTION CARDIAQUE

(30) Priorität: 27.05.1993 DE 4317752
(43) Veröffentlichungstag der Anmeldung: 13.03.1996
(73) Patentinhaber: FRAUNHOFER-GESELLSCHAFT ZUR FÖRDERUNG DER ANGEWANDTEN FORSCHUNG E.V., 80636 München (DE); FEINDT, Peter, D-66424 Homburg/Saar (DE); GAMS, Emmeran, D-66242 Homburg/Saar (DE)
(72) Erfinder: FEINDT, Peter, D-66424 Homburg/Saar (DE); GAMS, Emmeran, D-66242 Homburg/Saar (DE); STRAUB, Uwe, D-55743 Idar-Oberstein (DE); KAZI, Arif, D-38108 Braunschweig (DE)
(74) Vertreter: Münich, Wilhelm, Dr.
(86) Internationale Anmeldenummer: DE9400605
(87) Internationale Veröffentlichungsnummer: WO9427552

(56) Entgegenhaltungen:
- EP-A- 0 280 301
- DE-A- 3 307 211
- US-A- 3 478 737
- US-A- 4 304 225
- US-A- 4 690 134
- US-A- 5 131 905

## Beschreibung

### Technisches Gebiet

Die Erfindung bezieht sich auf eine Vorrichtung zur Unterstützung der Herzmuskelfunktion.

### Stand der Technik

Bei dem Pumpversagen des Herzens handelt es sich um eine muskuläre Schwäche der linken Herzkammer, durch die nicht genügend Blutvolumen vom Herzen in die Hauptschlagader ausgeworfen werden kann, so daß die Aufrechterhaltung des Kreislaufs nicht mehr gewährleistet ist. Die Ursachen für dieses Pumpversagen des Herzmuskels sind vielfältig; beispielsweise sind zu nennen: Viruserkrankungen, Minderdurchblutung des Muskels, Pumpversagen nach herzchirurgischen Eingriffen etc..

Bisherige Ansätze zur Behandlung dieser in den meisten Fällen zum Tode führenden Funktionsstörung des Herzens sind
1. medikamentöse Behandlung
2. Behandlung mittels einer intraaortalen Ballonpumpe
3. durch die Herztransplantation
4. durch einen sogenannten Muskelventrikel,
5. durch mechanische Pumpsysteme, die anstelle der linken Herzkammer in den Kreislauf ein geschaltet werden, wobei am Ende dieser Entwicklungen das sogenannte Kunstherz stehen soll.

Eine medikamentöse Beherrschung des Pumpversagens des Herzens ist nur bei temporären Schäden möglich, da aufgrund der hohen Nebenwirkungen diese starken Medikamente nur kurzzeitig zum Einsatz kommen können. Vor allem aber sind diese Medikamente nur für leichtere Formen des Pumpversagens einsetzbar, da auch hier nur eine unterstützende Wirkung auf den Herzmuskel zu erreichen ist, und ein manifest nicht mehr kontrahierender Muskel auch durch Medikamente nicht dazu zu veranlassen ist. Die Steigerung der Herzleistung beträgt dabei nur ca. 15%.

Die intraaortale Ballongegenpulsation, auch kurz IABP genannt, die 1968 eingeführt wurde, ist eine Methode, bei der ein Ballonkatheter in die Körperhauptschlagader eingeführt wird. Durch rhythmisches Aufblasen und Ablassen des Ballons erfolgt eine Erhöhung des diastolischen Blutflusses in den Herzkranzarterien; durch das erhöhte Angebot an Blut und die bessere Durchblutung des Herzmuskels wird eine Erhöhung der Pumpfunktion der linken Herzkammer erreicht. Bei dieser Methode kann nur eine Verbesserung um ca. 10 bis 20% erreicht werden. Die Nachteile dieser Methode sind zum einen die nur minimale Verbesserung der kardialen Pumpfunktion, zum anderen auch die Komplikationsmöglichkeiten, die hauptsächlich in Blutungen und Gefäßverschlüssen bestehen. Außerdem muß aufgrund des Fremdmaterials in einem Körpergefäß das Blut ungerinnbar gemacht werden.

Ein weiterer Ansatz zur Behandlung ist die Herztransplantation. Aufgrund von Berichten der Deutschen Gesellschaft für Thorax-Herz- und Gefäßchirurgie ist die Zahl der Transplantationen in der Bundesrepublik Deutschland seit 1991 relativ konstant. Die Ursache hierfür dürfte sein, daß nicht genügend Spenderherzen zur Verfügung stehen. Die Zahl der auf eine Transplantation Wartenden vermehrt sich jedoch jährlich um ca. 10%, ohne daß für dieses Problem eine Lösung gefunden werden könnte.

Vor allem aber können bei Herztransplantationen in der Regel nur Patienten berücksichtigt werden, die ein chronisches Pumpversagen haben, temporäre Versagen können mit dieser Methode praktisch nicht behandelt werden.

Weitere Nachteile von Herztransplantationen sind die hohen Sach- und Personalkosten. Auch müssen sich transplantierte Patienten einer lebenslangen Immunsuppression unterziehen, was zu einer erhöhten Infektanfälligkeit führt. Zusätzlich sind häufige stationäre Behandlungen mit Kontrollen des eingepflanzten Organs notwendig, die sehr zeit- und kostenaufwendig sind.

Letztlich weiß man, nachdem sich diese Methode nun seit einigen Jahren in der Klinik etabliert hat, daß sich aufgrund von Transplantatabstoßungen und von arteriosklerotischen Veränderungen der Herzkranzarterien dieser transplantierten Organe nur unbefriedigende Langzeitergebnisse erzielen lassen, außerdem ist das Auftreten von Tumoren bei diesen Patienten erhöht (Immunsuppression!).

Bei dem sogenannten Muskelventrikel handelt es sich um ein Operationsverfahren, bei dem der große Rückenmuskel ab-und freipräpariert wird, dann in den Brustkorb hinein und dort um das Herz herumgeschlagen wird. Vorher wird dieser Rückenmuskel durch elektrische Geräte induziert zum permanenten Schlagen angeregt, was eine Veränderung der Muskelfasern nach sich ziehen soll. Nachdem dieser Rückenmuskel dann um das Herz herumgewickelt und festgenäht ist, und er sich rhythmisch kontrahiert, wird das Herz zusammengedrückt und so ein erhöhtes Blutauswurfvolumen gefördert. Dieser Ansatz, der sich noch im Experimentierstadium befindet, weist ebenfalls eine große Zahl von Nachteilen auf:
1. Derzeit gelingt es noch nicht vollständig und vor allem nicht für genügend lange Zeit, durch die elektrischen Impulse eine Umformung der Muskelfasern zu erreichen.
2. Bei dieser Methode wird immer das gesamte Herz komprimiert, also auch Bereiche, die physiologisch eigentlich nicht komprimiert werden sollten.
3. Bei dieser Methode ist eine sehr große Operation notwendig, so daß temporäres Pumpversagen mit dieser Methode nicht behandelt werden können.

Letztlich wird schon seit Jahren der Versuch unternommen, ein Kunstherz zu entwickeln, das nach Entfernung des körpereigenen Herzens an die gleiche Stelle implantiert und in den Kreislauf eingeschaltet wird. Dieses Vorhaben scheitert bislang jedoch an den bisher zur Verfügung stehenden Materialien, die zur Zeit noch eine sehr geringe Verträglichkeit mit den Körpergeweben und vor allen Dingen mit dem körpereigenen Blut aufweisen. Durch den Kontakt des Blutes mit körperfremden Oberflächen kommt es zu schweren Eingriffen in das Gerinnungssystem, mit daraus folgenden möglichen Blutungen. Auch die medikamentöse Beeinflussung des Blutes mittels Heparin, das das Blut ungerinnbar macht, gibt hier im Langzeitverlauf keinen Schutz, immer wieder gehen Blut bzw. Blutbestandteile zugrunde, so daß dem Patienten in regelmäßigen Zeitabständen immer wieder Fremdblut (Übertragung von Viruserkrankungen) substituiert werden muß. Parallel zu dieser Entwicklung des Kunstherzens wurden Unterstützungssysteme entwickelt, die unter Belassung des Herzens temporär in den Blutkreislauf eingesetzt werden können. All diesen Systemen ist jedoch gemeinsam, daß sie ebenfalls mit dem Blut des Patienten in Kontakt kommen und so zu einer Aktivierung der Blutgerinnung an der fremden Oberfläche führen. Zusätzlich müssen künstliche Blutgefäße zu diesen Pumpsystemen geführt und von ihnen wieder zurück in den Körper implantiert werden, bei dem Durchtritt dieser körperfremden Anschlüsse müssen diese nach auβerhalb des Körpers austreten und bergen so eine hohe Gefahr an Infektionen.

In der US-PS 4 690 134 und in der DE-OS 33 07 211 werden Vorrichtungen zur Unterstützung der Herzfunktion vorgestellt. In beiden Dokumenten werden Systeme des Myocard im Sinne eines Behältergehäuses umfassen. Dabei kommt es allerdings zu einem gleichmäßigen Druck auf das gesamte rechte Niederdrucksystem des Herzens (rechter Vorhof und rechter Ventrikel) und somit zu völlig unphysioligischen Drücken von über 100mmHg. Dieses ist für eine Zeit von mehr als 5 Minuten von keinem Herzen ohne Schäden auszuhalten.

Auch die US 4 304 225 beschreibt eine Vorrichtung zur Unterstützung der Herzfunktion. In der EP 0 280 301 wird eine Vorrichtung beschrieben, bei der durch drei plane Platten zumindest indirekt, wenn nicht sogar direkt auf den rechten Ventrikel gedrückt wird. Durch die pumpförmige Druckbeaufschlagung wird das Herz weggedrückt. Der direkte Druck auf das Herz führt zu Schäden.

### Darstellung der Erfindung

Der Erfindung liegt die Aufgabe zugrunde, eine Vorrichtung anzugeben, die insbesondere bei temporärem Pump-versagen die Herzfunktion wirkungsvoll unterstützen kann.

Eine erfindungsgemäße Lösung dieser Aufgabe ist im Patentanspruch 1 angegeben. Weiterbildungen der Erfindung sind Gegenstand der Unteransprüche.

Die Erfindung geht von der Idee aus, das erkrankte Herz und hier speziell die linke Herzkammer durch mechanischen Druck auf dieselbe zu unterstützen, um so den Blutauswurf in die Hauptschlagader zu erhöhen und einen normalen Kreislauf aufrechtzuerhalten.

Hierzu weist die erfindungsgemäße Vorrichtung folgende Merkmale auf:
- mindestens eine Schale, deren Form an die Form des menschlichen Herzens angepaßt ist, und die in den menschlichen Brustkorb derart eingesetzt werden kann, daß sie das Herz mit Ausnahme der zum Herz führenden und vom Herz wegführenden Adern mindestens teilweise umschließt,
- mindestens eine Füllkammer, die sich in oder an der bzw. den Schalen befindet, und die sich an der Schale abstützt,
- einen pneumatischen Antrieb, der ein Gas in die Füllkammer(n) füllen kann, und
- eine Steuereinheit mit Sensoren, die die Herzaktion erkennen, und die den Antrieb synchron zur Herztätigkeit steuert,

Die Erfindung ist dadurch gekennzeichnet, daß das Gas Luft ist, daß die Füllkammer(n) jeweils in mehrere Füllkammerelemente unterteilt sind, und derart in bzw. an der Schale/ an den Schalen im Bereich der linken Herzkammer angeordnet sind, daß sie Druck ausschließlich auf die linke Herzkammer ausüben können und ein Druckaufbau an den Seiten des Herzmuskels vermieden wird, so daß der Druckanstieg im rechten Ventrikel so gering wie möglich gehalten wird, und daß der Druckaufbau in den Füllkammerelementen zeitlich versetzt derart erfolgt, daß der Druckaufbau an der Herzspitze beginnt.

Bei der erfindungsgemäßen Vorrichtung wird die Auswurfleistung des Herzens durch einen genau definierten Druck auf die linke Herzkammer gesteigert. Der dabei entstehende Gegendruck wird durch entsprechend gefüllte Kammern gleichmäßig verteilt. Durch eine Abpolsterung des Herzmuskels wird der Druck dabei gleichmäßig verteilt, so daß eine Verletzung des Herzmuskels und der Herzkranzgefäße vermieden wird. Die Aktion des Systems wird durch eine Koppelung der elektrischen Herzaktion mit der mechanischen Herzaktion synchronisiert.

Die Energiequelle des Systems sowie ihre Steuereinheit und der Antrieb sind vollständig in den Körper implantierbar. Da bei den meisten Herzerkrankungen, die zu einer eingeschränkten Pumpfunktion führen, auch der Herzrhythmus gestört ist, ist es von Vorteil, wenn die Steuereinheit einen Herz-Schrittmacher aufweist.

Als Vorteil der erfindungsgemäßen Vorrichtung gegenüber den bisher bekannten Lösungen ist der völlig neue Ansatz:

Das erkrankte Herz wird nicht aus dem Kreislauf ausgeschaltet, sondern es wird durch die erfindungsgemäße Vorrichtung unterstützt.

Somit entfällt, daß das Blut des Patienten mit körperfremden Oberflächen in Kontakt kommt; auch die Probleme, die unter dem Oberbegriff Biokompatibilität zusammengefaßt werden, treten nicht auf. Ein weiterer großer Vorteil ist, daß sämtliche Teile des Systems in den Körper implantiert werden und durch den fehlenden Durchtritt von Leitungen die Infektionsgefahr auf ein Minimum reduziert wird. Daraus resultiert ebenfalls, daß die Patienten nicht stationär im Krankenhaus gehalten werden müssen, sondern sie völlig frei und mobil sind und so eine enorme Senkung der Kosten vorgenommen werden kann.

Die erfindungsgemäße Vorrichtung kann bei allen temporären oder manifesten Zustandsformen angewendet werden, bei denen der linke (oder auch der rechte) Ventrikel aufgrund einer myokardialen Schwäche bzw. Funktionsstörung nicht in der Lage ist, ein zur Aufrechterhaltung des Kreislaufs genügend großes Schlagvolumen (Blutvolumen) mit ausreichendem Druck in die Aorta zu pumpen. Diese Funktionsstörung ist dabei völlig unabhängig von der Ursache.

Ausgelegt werden kann das System zunächst auf kurzzeitige Anwendungen, bei guter Verträglichkeit können jedoch auch Pumpfunktionsstörungen des Herzens angegangen werden, die einer Langzeitanwendung bedürfen, so z.B. alle Patienten, die zu einer Herztransplantation anstehen.

Ein weiterer Vorteil des Systems liegt darin, daß das System anders als die meisten anderen zur Zeit angewendeten bei Besserung der eigenen Herzfunktion, die bei kurzzeitigen Anwendungsindikationen zu erwarten ist, langsam reduziert und ausgeschlichen werden kann.

Die Patienten sind zusätzlich mobil, müssen nicht stationär behandelt werden, womit eine drastische Kosteneinsparung erfolgt.

Bei der in den Brustkorb zu implantierenden Vorrichtung handelt es sich bevorzugt um doppelwandige Hartschalen in die das Herz hineingelegt wird und die im Bereich des Austritts der großen Gefäße aus dem Herz befestigt werden können. Es sind natürlich auch andere Befestigungsmöglichkeiten denkbar. Insbesondere ist es möglich, daß die Hartschale bzw. die Hartschalen mit Verbindungsstücken derart ausgeführt sind, das keine weitere Halterung benötigt wird, das sich die Vorrichtung also selbst um das Herz hält. Insbesondere ist die Ausführungsform bei Herzmuskelfunktionsunterstützungsvorrichtungen, die für eine längere Zeit sich im Körper des Patienten befinden soll, vorzuziehen. Diese selbsthaltende Vorrichtung wird also dann zu bevorzugen sein, wenn der Brustkorb nach der Operation wieder geschlossen wird.

Zur Sicherung gegen ein Abrutschen der Kompressionseinheit in Richtung der Herzachse läßt sich ein Riemen um Vorhöfe und Blutgefäße herumführen. Die resultierende Druckkraft in Richtung der Herzachse ist allerdings nicht zu vernachlässigen, d.h. daß es selbst bei einer relativ großen Auflagefläche des Riemens zu einer Einschnürung der Blutgefäße und einer Behinderung des Blutflusses kommen kann.

Riemen an den offenen Seiten der Kompressionseinheit können dazu dienen, eine seitliche Verlagerung der Kompressionseinheit gegenüber dem Herzmuskel zu verhindern. Es bietet sich an, die Riemen in Höhe der Ventilebene anzubringen, da dort keine großen Verformungen des Herzmuskels erfolgen. Allerdings bewegt sich die Ventilebene während der Herzkontraktion, d.h. die Riemen sollten aus einem möglichst weichen Material bestenachvollziehen zu können.

Weitere Befestigungsmöglichkeiten sind z. B. die Befestigung der Kompressionseinheit am Herzbeutel unterhalb des Herzmuskels oder an den Seiten oder eine Fixierung am Brustkorb.

Die äußere Wand der Schale besteht aus einem festen Material, das nicht elastisch ist und den anatomischen Gegebenheiten angepaßt wird. Die innere Schicht der Schale besteht aus einem elastischen Kunststoff ähnlichen Material und ist in mehreren Kammern unterteilt.

Die Druckausübung auf die linke Herzkammer geschieht durch pneumatisch angetriebenes Aufblasen einzelner oder mehrerer Kammern, die über der linken Herzkammer, diese zusammendrücken und somit zu einem Auswurf von Blut in die Körperschlagader führen. Der dabei auftretende Gegendruck wird durch Füllung von der linken Herzkammer gegenüberliegender Luftkammern aufgefangen. Der Antrieb des Systems und die Steuerung erfolgen über zwei getrennte Systeme, die nach bewährter Methode subkutan implantiert werden und mit Leitungssystemen mit der um das Herz stitzenden Schale bzw. den um das Herz sitzenden Schalen verbunden werden. Das Steueraggregat wird zusätzlich mit einem Herzschrittmachersystem gekoppelt, um so bei einem eventuell ausfallenden Herzeigenrhythmus, der aufgrund vieler Grunderkrankungen, die zu einem Pumpversagen führen, häufig vorkommt, unabhängig zu sein. Über das Steuerungssystem erfolgt durch Sensoren eine Erkennung der Herzaktion und durch Triggern dieses Signals wird das Kammersystem während der Herzsystole pneumatisch angetrieben aufgeblasen. Die Stärke des Aufblasens und die Verteilung auf die einzelnen Kammern sowie die zeitlichen Komponenten sind dabei über bereits verfügbare telemetrische Systeme von außen zu steuern, eine Anpassung an die akute Herzeigenarbeit ist somit gewährleistet.

Die Kontraktion des Herzmuskels des Herzens an sich beginnt an der Herzspitze und setzt sich zur Ventilebene hin fort. Der zeitliche Verlauf der Druckbeaufschlagung sollte schon aus physiologischen Gründen in der gleichen Weise erfolgen. Die natürliche Herzbewegung in strömungstechnischer Hinsicht erscheint genauso weitgehend optimal, da das Blut in den Ventrikeln bei einer an der Herzspitze beginnenden Kontraktion von Beginn an in Richtung der Ventile beschleunigt wird. Am Ende der Systole muß für einen schnellen Abbau des aufgebrachten Drucks gesorgt werden, damit das Füllen der Ventrikel nicht behindert wird. Eine derartige Druckbeaufschlagung kann durch die erfindungsgemäße Vorrichtung über eine entsprechende Steuerung erfolgen. Der notwendige zusätzliche Druck zum außen herrschenden Druck, der auf den Herzmuskel ausgeübt werden muß bestimmt sich durch den systolischen Blutdruck im linken Ventrikel. Da dieser ungefähr 160 mbar ist, sollte ein äußerer Überdruck auf die Herzwand von 200 mbar ausreichend sein. Auf jeden Fall ist dabei ein Überdruck von 300 mmbar ausreichend.

Der Druck in der Kompressionseinheit sollte derart angelegt werden, daß der Druckaufbau bei der Herzspitze anfängt und sich von der Herzspitze wegbewegt. Kurz bevor der Druck wieder abgebaut wird haben die Druckkammern der Kompressionseinheit einen gleichmäßig großen Druck. Wenn der Druck voll aufgebaut ist, d.h. er bei der Systole angelangt ist, dann sollte der Druck möglichst schnell abgebaut werden. Dieses geschieht durch Beaufschlagung der Kompressionseinheit mit typischerweise 100 mbar unter dem Umgebungsdruck.

Die Kompressionseinheit sollte, sofern sie auch für längere Zeit im Körper des Patienten bleiben soll, symmetrisch aufgebaut sein, da der Betrieb des Systems unabhängig von der Körperlage des Patienten erfolgen muß. Ein symmetrischer Aufbau in diesem Sinne bedeutet, daß gegenüberliegend entsprechend gleich viele Füllkammern vorgesehen sind.

Die Verzögerung des Druckaufbaus kann durch eine entsprechende konstruktive Gestaltung der Füllkammern realisiert werden. Es bietet sich an, die Füllkammern auf beiden Seiten des Herzmuskels zu unterteilen und die Füllkammerelemente zeitlich versetzt mit Druck zu beaufschlagen. Bei einer Reihenschaltung von mehreren Füllkammerelementen, ist die Zeitverzögerung des Druckaufbaus durch zusätzliche Elemente, die die Luftzufuhr zu den einzelnen Füllkammerelementen beeinflussen, optimal zu den gegebenen Bedingungen anzupassen. Die Realisierung der Zeitverzögerung durch eine Reihenschaltung von Füllkammerelementen hat sicherlich deutliche Vorteile gegenüber einer entsprechenden konstruktiven Gestaltung der Füllkammer, da eine variablere Steuerung ermöglicht wird.

Die typischen Parameter für die Druckerzeugungseinheit, die typischerweise bei Patienten verwendet werden sind z.B. der Druckaufbau von 200 mbar in 60ms und einer mittleren Fördervolumenstrom von Vₘᵢₜₜₑₗ = 200 cm³/s.

Es kann auch ein maximaler Fördervolumenstrom von Vₘₐₓ = 1000 cm³/s erreicht werden.

Zusammenfassend sind als Vorteile zu nennen:

Zunächst einmal wird das erkrankte Organ Herz nicht aus dem Kreislauf herausgenommen, vielmehr wird versucht, die fehlende Kontraktionskraft durch Druck von außen zu ersetzen und so das Herz in seiner Funktion zu unterstützen. Damit verbunden ist die Tatsache, daß an keiner Stelle des Körpers Blut mit körperfremden Oberflächen in Kontakt gerät und damit sämtliche schweren Probleme der Biokompatikbilität entfallen. Zusätzlich sind alle Teile des Systems vollständig in den Körper zu implantieren, so daß auch die Gefahr von Infektionen auf ein Minimum herabgesetzt wird. Die Patienten sind somit mobil, müssen nicht stationär intensiv-medizinisch überwacht werden, was zusätzlich eine drastische Kosteneinsparung bedeutet. Eine Blutverdünnung muß damit ebenfalls nicht erfolgen. Durch die von außen zu steuernde Aufblasbarkeit der einzelnen Kammer des sackartigen Gebildes erfolgt eine flexible Anpassung des Systems an die noch vorhandene Herzeigenarbeit, womit eine eventuell langsame Reduzierung und Anpassung erfolgen kann.

Ausgelegt werden soll das System zunächst auf kurzzeitige Anwendungsindikationen, bei guter Verträglichkeit können dann jedoch durch chronische Pumpfunktionsstörungen des Herzens behandelt werden, womit dann eine Vielzahl von Patienten zu behandeln wären, die heute noch vergeblich auf eine Herztransplantation warten müssen.

Durch die erfindungsgemäße Vorrichtung ist es möglich, den linken Ventrikel des Herzens bei minimaler Druckerhöhung im rechten Ventrikel zu unterstützen. Eine Steigerung der Herzleistung von mindestens 25 % ist wünschenswert. Eine weitere erfindungsgemäße Fortbildung der Vorrichtung sieht vor, den Betrag der Unterstützung zwischen 0 und 100 % einzustellen. Dazu wird die verwendete Steuereinheit zusammen mit der Druckerzeugungseinheit und/oder der Kompressionseinheit derart verwendet, daß der linke ventrikel des Herzens unterschiedlich starken Drücken ausgesetzt wird.

### Kurze Beschreibung der Zeichnung

Die Erfindung wird nachstehend ohne Beschränkung des allgemeinen Erfindungsgedankens anhand von Ausführungsbeispielen unter Bezugnahme auf die Zeichnung exemplarisch beschrieben, auf die im übrigen bezüglich der Offenbarung aller im Text nicht näher erläuterten erfindungsgemäßen Einzelheiten verwiesen wird. Es zeigen:
- Fig. 1: ein Herz mit der erfindungsgemäßen Vorrichtung im Querschnitt durch den linken Ventrikel,
- Fig. 2: eine Seitenansicht der erfindungsgemäßen Vorrichtung,
- Fig. 3: eine Vorderansicht der erfindungsgemäßen Vorrichtung,
- Fig. 4: eine Füllkammer aus mehreren, übereinander angeordneten Kammerelementen, und
- Fig. 5: eine funktionsfähige Kompressionseinheit. Links im nicht aufgeblasenen Zustand und rechts im aufgeblasenen Zustand.

### Beschreibung von Ausführungsbeispielen

In den folgenden Figuren sind jeweils gleiche oder entsprechende Teile mit den selben Bezugszeichen bezeichnet, so daß auf eine erneute Vorstellung verzichtet wird, und lediglich die Abweichungen der in diesen Figuren dargestellten Ausführungsbeispiele gegenüber dem ersten Ausführungsbeispiel erläutert werden:

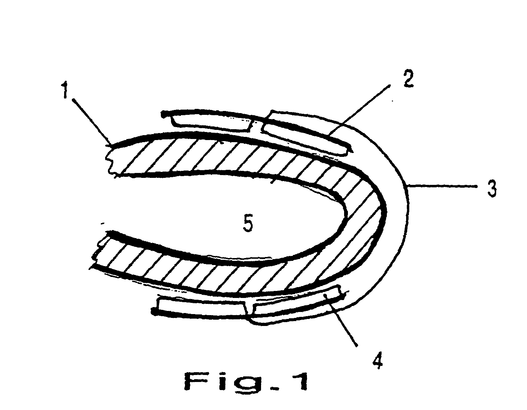

In Figur 1 wird eine linke Herzkammer (1) und die erfindungsgemäße Vorrichtung im Querschnitt gezeigt. Links ist auf dieser Zeichnung kranial, rechts sandal, oben ventral und unten dorsch. Die erfindungsgemäße Vorrichtung besteht aus Hartschalen (2), die durch ein Verbindungsstück (3) verbunden sind. Füllkammern (4) sind auf den Hartschalen angebracht und zwar derart, daß sie zum Herz (1) gerichtet sind.

Die Hartschalen (2) und das Verbindungselement (3) sollte eine hohe Verformungssteifigkeit besitzen, um ein Ausweichen der Füllkammern nach außen zu vermeiden. Das Verbindungselement (3) sollte dabei verstellbar sein, um eine Anpaßbarkeit an den Herzmuskel zu gewährleisten.

Die Geometrie des Verbindungselements (3) muß den räumlichen Gegebenheiten Rechnung tragen. Eine Verbindung an der Herzbasis ist aufgrund der schlechten Zugänglichkeit wegen dort vorhandener Blutgefäße z. B. weniger geeignet. Deshalb wird eine bevorzugte Verbindung an den Seiten des Herzmuskels oder an der Herzspitze vorgesehen.

Ein Druckaufbau an den Seiten des Herzmuskels sollte vermieden werden, um den Druckanstieg im rechten Ventrikel so gering wie möglich zu halten. Besonders bevorzugt ist deswegen die Plazierung des Verbindungselementes an der Herzspitze, weil diese während der Herzkontraktion weitgehend stationär bleibt.

Die Füllkammern (4) werden im gewünschten Rhythmus mit Luft gefüllt und wieder entleert, um so die Muskeln des linken Ventrikels (5) zu unterstützen. Damit kommt es zu einer Unterstützung der myokardialen Kontraktionskraft, also zu einer Unterstützung der Herzfunktion im allgemeinen.

Der Auffang des Gegendruckes durch Aufblasen der Füllkammern (4) erfolgt durch Aufblasen von gegenüberliegenden Füllkammern (4).

Der Einfachheithalber wird die erfindungsgemäße Vorrichtung im folgenden IVAD genannt, was sich aus intrathoracic ventricular assist device herleitet.

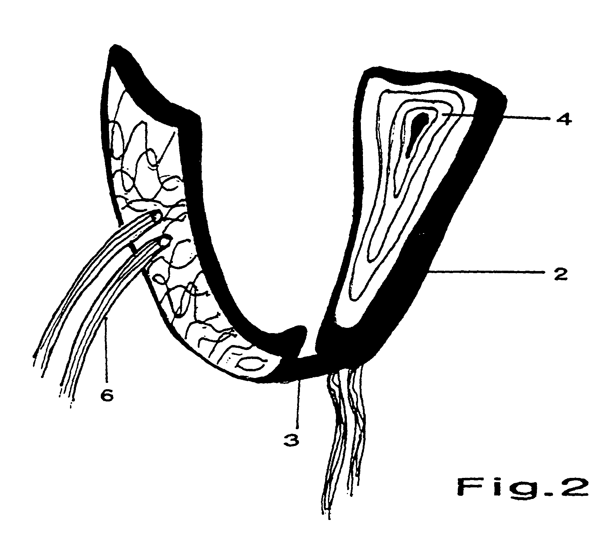

Figur 2 zeigt eine Seitenansicht des IVAD. Gut zu erkennen sind die Gasleitungen (6), die zu den Füllkammern (4) führen. Damit ist es möglich, die Luft in die Füllkammern (4) zuzuleiten bzw. abzuführen.

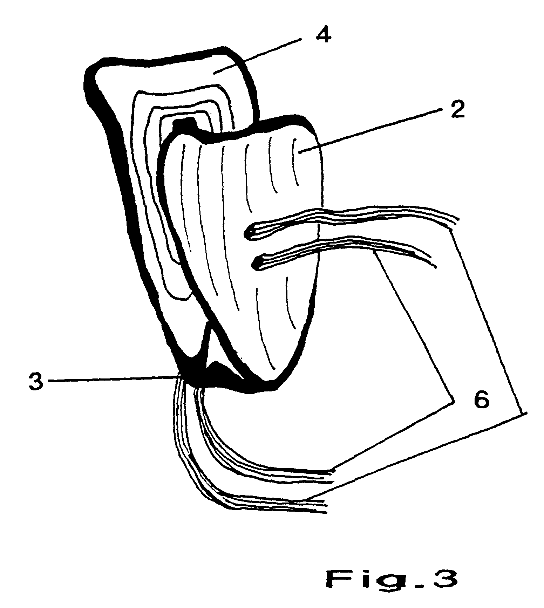

Figur 3 zeigt eine Vorderansicht des IVAD. Die Hartschalen sind der Form des Herzens (1) angepaßt und bestehen vorzugsweise aus einem körperverträglichen Material.

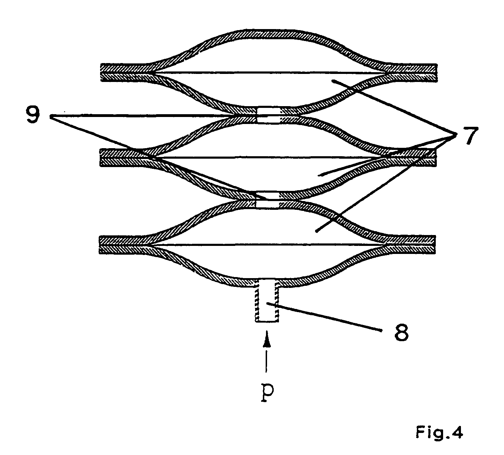

Figur 4 zeigt eine Füllkammer aus mehreren, übereinander angeordneten Kammerelementen (7). Die Kammerelemente (7) bestehen aus biokompatiblem Material, wie z. B. Silikonkautschuk, Polytetrafluorethylen (PTFE, 'Teflon') oder Polyurethan (PU). Das beste Material wäre ein solches, daß sehr dehnfähig ist, eine geringe Stärke hat, sehr fest und deren Dehnung reversibel verläuft. Es hat sich herausgestellt, daß Polyurethan als Material für Kammerelemente (7) am besten geeignet ist.

Die verwendeten Folien aus z.B. PU können an den Enden entweder thermisch zusammengeschweißt oder verklebt werden. In Figur 4 ist zusätzlich noch ein Füllrohr (8) zu sehen, an dem die Gasleitungen (6) angebracht werden. Die einzelnen Kammerelemente (7) der Füllkammer (4) sind durch Öffnungen (9) miteinander derart verbunden, daß ein Druckausgleich über die jeweiligen Kammerelemente stattfinden kann.

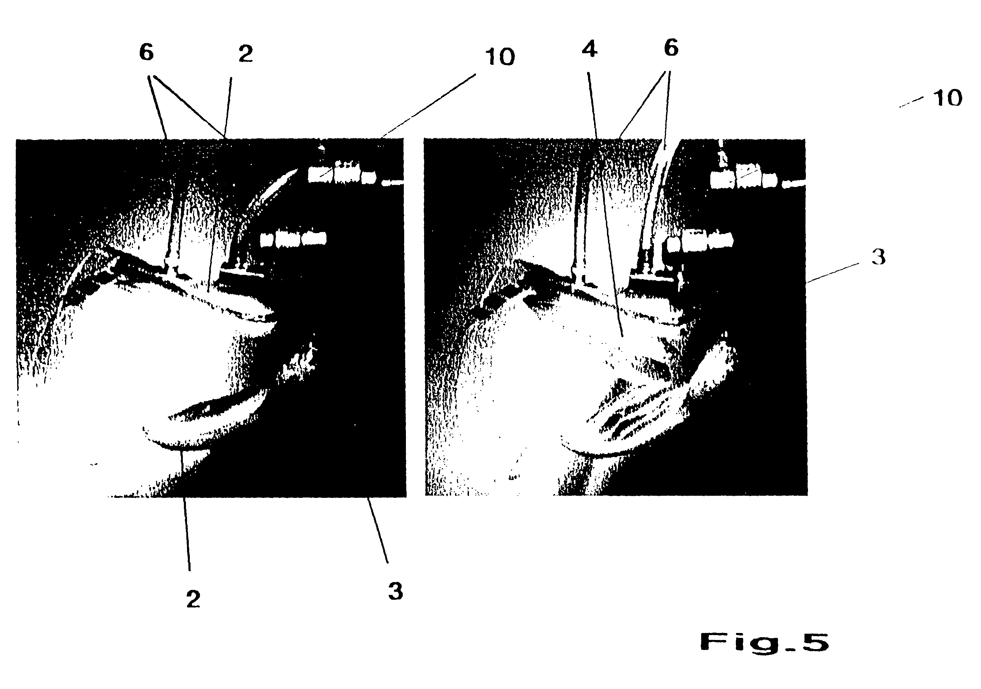

In Figur 5 ist eine funktionsfähige Kompressionseinheit zu sehen, die für Vorexperimente genutzt wurde. Auf der linken Seite der Figur 5 ist die Kompressionseinheit in nicht aufgepumptem Zustand zu erkennen und auf der rechten Seite ist die Kompressionseinheit aufgepumpt. Das zusätzliche Gestänge (10) diente bei den Vorversuchen zur Befestigung der Kompressionseinheit an der Toraxklammer, die den Brustkorb auseinandergehalten hat. Als Schalenmaterial der Hartschalen (2) werden typischerweise auch biokompatible Materialien wie z. B. Polymethylmethacrylat (PMMA,'Plexiglas') oder glasfaserverstärkter Kunststoff auf Epoxidbasis verwendet.

## Patentansprüche

1. Vörrichtung zur Unterstützung der Herzfunktion, mit
- mindestens einer Schale (2,3), deren Form an die Form des menschlichen Herzens (1) angepaßt ist, und die in den menschlichen Brustkorb derart eingesetzt werden kann, daß sie das Herz mit Ausnahme der zum Herz führenden und vom Herz wegführenden Adern mindestens teilweise umschließt,
- mindestens einer Füllkammer (4), die sich in oder an der bzw. den Schalen (2) befindet, und die sich an der Schale (2) abstützt,
- einem pneumatischen Antrieb (6), der ein Gas in die Füllkammer(n) füllen kann, und
- einer Steuereinheit mit Sensoren, die die Herzaktion erkennen, und die den Antrieb synchron zur Herztätigkeit steuert,
**dadurch gekennzeichnet, daß** das Gas Luft ist, daß die Füllkammer(n) jeweils in mehrere Füllkammerelemente unterteilt sind, und derart in bzw. an der Schale/ an den Schalen im Bereich der linken Herzkammer angeordnet sind, daß sie Druck ausschließlich auf die linke Herzkammer ausüben können und ein Druckaufbau an den Seiten des Herzmuskels vermieden wird, so daß der Druckanstieg im rechten Ventrikel so gering wie möglich gehalten wird, und daß der Druckaufbau in den Füllkammerelementen zeitlich versetzt derart erfolgt, daß der Druckaufbau an der Herzspitze beginnt.

2. Vorrichtung nach Anspruch 1,
**dadurch gekennzeichnet, daß** die Schalen als Hartschalen ausgebildet sind, die für die verwendeten Kräfte nicht deformierbar sind.

3. Vorrichtung nach Anspruch 1 oder 2,
**dadurch gekennzeichnet, daß** der Antrieb des Systems und die Steuereinheit subkutan implantiert sind.

4. Vorrichtung nach einem der Ansprüche 1 bis 3,
**dadurch gekennzeichnet, daß** die Steuereinheit extrakorporal angeordnet ist, und den Antrieb über ein Telementriesystem steuert.

5. Vorrichtung nach einem der Ansprüche 1 bis 4,
**dadurch gekennzeichnet, daß** der beim Drücken auf eine Herzkammer entstehende Druck durch eine entsprechende Füllung der gegenüberliegenden Kammer(n) aufgefangen wird.

6. Vorrichtung nach einem der Ansprüche 1 bis 5,
**dadurch gekennzeichnet, daß** die Steuereinheit und der Antrieb zweifach vorhanden sind.

7. Vorrichtung nach einem der Ansprüche 1 bis 6,
**dadurch gekennzeichnet, daß** die Schalen derart geformt sind, daß das Herz im Bereich des linken Ventrikels von den Schalen mindestens teilweise umschlossen ist.

## Claims

1. Device for supporting the cardiac function, comprising
- at least a shell (2, 3) whose shape is adapted to the shape of the human heart (1) and that is adapted for being implanted into the human thorax in such a way that it encloses the heart, at least partly, with the exception of the blood vessels leading to and away from the heart,
- at least one filling chamber (4) that is provided in or on said shell or shells (2), respectively, and is supported on said shell (2),
- a pneumatic drive (6) adapted to charge said filling chamber(s) with a gas, and
- a controller unit including sensors detecting the cardiac action and controlling said drive in synchrony with the cardiac activity,
**characterised in that** said gas is air, that each of said filling chamber(s) is/are subdivided into several filling chamber elements and arranged in or on said shell/on said shells in the region of the left ventricle in such a way that they can pressurise the left ventricle exclusively and that the production of pressure on the sides of the cardiac muscle is avoided so as to maintain the pressure increase in the right ventricle as small as possible, and that the production of pressure in said filling chamber elements takes place with such a time offset that the pressure production begins at the apex of the heart.

2. Device according to Claim 1,
**characterised in that** said shells are configured as hard shells which cannot be deformed for the applied forces.

3. Device according to Claim 1 or 2,
**characterised in that** said drive of the system and said controller unit are implanted subcutaneously.

4. Device according to any of the Claims 1 to 3,
**characterised in that** said controller unit is disposed outside the body and controls the drive via a telemetric system.

5. Device according to any of the Claims 1 to 4,
**characterised in that** the pressure created by pressing on a ventricle is absorbed by an appropriate filling in the opposite chamber(s).

6. Device according to any of the Claims 1 to 5,
**characterised in that** said controller unit and said drive are provided in duplicate.

7. Device according to any of the Claims 1 to 6,
**characterised in that** said shells are so shaped that the heart is enclosed by said shells, at least partly, in the region of the left ventricle.

## Revendications

1. Dispositif d'assistance à la fonction cardiaque, comprenant
- au moins une coque (2, 3) dont la forme est adaptée à la forme du coeur humain (1) et qui est apte à être implantée dans le thorax humain d'une façon, qu'elle renferme le coeur, au moins en partie, à l'exception des vaisseaux sanguins menant au coeur et d'en sortant,
- au moins une chambre de remplissage (4), qui est disposée dans ou sur ladite coque ou respectivement lesdites coques (2), en étant appuyée sur ladite coque (2),
- une unité d'entraînement pneumatique (6) apte à remplir ladite chambre ou lesdites chambres de remplissage d'un gaz, et
- une unité de commande, qui comprend des palpeurs à détecter les fonctions cardiaques et à commander ladite unité d'entraînement de façon synchronisée avec le fonctionnement cardiaque,
**caractérisé en ce que** ledit gaz est de l'air, **en ce que** ladite chambre de remplissage ou chacune desdites chambres de remplissage est/sont divisée(s) en une pluralité d'éléments de chambre de remplissage et disposée(s) dans ou sur ladite coque/sur lesdites coques dans la zone du ventricule gauche exclusivement, et **en ce que** l'établissement de pression sur les côtés du myocarde est empêché afin de tenir la montée de la pression dans le ventricule droit au plus bas niveau possible, et **en ce que** la création de la pression dans lesdits éléments de chambre de remplissage se fait à un tel déport en temps, que l'établissement de pression commence à la pointe du coeur.

2. Dispositif selon la revendication 1,
**caractérisé en ce que** lesdites coques sont configurées sous forme de coques dures, qui ne sont pas déformables pour les efforts appliqués.

3. Dispositif selon la revendication 1 ou 2,
**caractérisé en ce que** ladite unité d'entraînement du système et ladite unité de commande sont implantées de façon sous-cutanée.

4. Dispositif selon une quelconque d es revendications 1 à 3,
**caractérisé en ce que** ladite unité de commande est disposée à l'extérieur du corps et commande l'unité d'entraînement via un système télémétrique.

5. Dispositif selon une quelconque des revendications 1 à 4,
**caractérisé en ce que** la pression engendrée en pressant sur un ventricule est absorbée par un intérieur approprié dans la chambre ou les chambres opposée(s).

6. Dispositif selon une quelconque des revendications 1 à 5,
**caractérisé en ce que** ladite unité de commande et ladite unité d'entraînement sont disposées en double.

7. Dispositif selon une quelconque des revendications 1 à 6,
**caractérisé en ce que** lesdites coques sont formées de façon, que le coeur soit renfermé par lesdites coques, au moins en partie, dans la zone du ventricule gauche.
